# EUROPÄISCHE PATENTANMELDUNG

(11) **EP 4 717 147 A2**
(43) Veröffentlichungstag der Anmeldung: **01.04.2026**
(21) Anmeldenummer: 26158687.9
(22) Anmeldetag: 17.06.2020
(51) Int. Cl.: A61B 1/05

(54) **INSTRUMENTENSCHAFT MIT MEHREREN KANÄLEN SOWIE DESSEN FERTIGUNG**

(30) Priorität: 19.06.2019 DE 102019116583
(62) Teilanmeldung aus: 20180638.7
(71) Anmelder: Karl Storz SE & Co. KG, 78532 Tuttlingen (DE)
(72) Erfinder: CZUPALLA, Christian, 78532 Tuttlingen (DE); LIMBERGER, Andreas, 78532 Tuttlingen (DE); ULMSCHNEIDER, Daniel, 78532 Tuttlingen (DE); KÖNIG-URBAN, Kamilla, 78532 Tuttlingen (DE)

(57) **Zusammenfassung**

Die Offenbarung bezieht sich auf ein medizinisches Instrument zur Bereitstellung eines Zugangs zum Körperinneren, insbesondere Spatel (12) für ein endoskopisches Instrument (10), mit einem Schaft (14) mit einem langgestreckten Grundkörper (16), wobei sich der Grundkörper (16) zwischen einem distalen Ende (30) und einem proximalen Ende (32) des Schaftes (14) erstreckt, und einem mit dem Grundkörper (16) des Schaftes (14) an dessen proximalem Ende (32) gekoppelten Griff (20), wobei der Schaft (14) zumindest einen ersten Kanal (40) und einen zweiten Kanal (42) definiert, die sich durch den Schaft (14) erstrecken, wobei der erste Kanal (40) eine Passage für chirurgische Instrumente durch den Schaft (14) hindurch bereitstellt, wobei der zweite Kanal (42) zur Aufnahme einer Beobachtungsoptik (46) ausgebildet ist, und wobei dem zweiten Kanal (42) zumindest ein Nebenkanal (78, 80) benachbart ist, der mit dem zweiten Kanal (42) verbunden ist. Die Offenbarung bezieht sich ferner auf ein Verfahren zur Herstellung eines solchen Instruments.

## Beschreibung

Die vorliegende Offenbarung befasst sich mit medizinischen Instrumenten und Bauteilen, insbesondere mit solchen, die zur Bereitstellung eines Zugangs zum Körperinneren genutzt werden, sowie mit deren Herstellung. Insbesondere, aber nicht ausschließlich, bezieht sich die vorliegende Offenbarung auf einen Spatel für ein endoskopisches Instrument, insbesondere auf einen Spatel für ein Mediastinoskop, beispielsweise in Form eines Video-Mediastinoskops.

Aus der US 2007/0106122 A1 ist ein Gerät für die endotracheale Intubation bekannt, mit einem Hauptkörper, der ein in den Körper einführbares spatelförmiges Instrument trägt, dass einen geraden und einen gekrümmten Abschnitt aufweist, an den sich eine distale Zunge anschließt, wobei ein offener und ein geschlossener Kanal vorgesehen ist. Das einführbare spatelförmige Instrument besteht aus einem Kunststoff.

Die vorliegende Offenbarung bezieht sich auf medizinische Instrument und Bauteile, die genutzt werden, um einem Operateur oder sonstigem medizinischen Personal einen Zugang zum Körperinneren bereitzustellen, beispielsweise auf Instrumente für die minimalinvasive Chirurgie bzw. für die Endoskopie. Zugänge zum Körperinneren können über natürliche oder künstlich geschaffene Öffnungen bereitgestellt werden.

Zumindest in beispielhaften Ausgestaltungen bezieht sich die vorliegende Offenbarung auf Instrumente und Geräte für die Mediastinoskopie. Die Offenbarung ist jedoch nicht ausschließlich hierauf beschränkt. Beispielhaft kann es sich allgemein um Instrumente für die Thoraxchirurgie oder allgemein um endoskopische Instrumente handeln.

Allgemein bezieht sich die Offenbarung auf solche endoskopischen Geräte und Baugruppen, die mehrere Kanäle in einem Schaft bereitstellen. Beispielsweise handelt es sich bei den zumindest zwei Kanälen um einen ersten Kanal zur Bereitstellung einer Passage für ein chirurgisches Instrument oder dergleichen, sowie um einen zweiten Kanal zur Aufnahme einer Beobachtungsoptik. Der zweite Kanal kann neben der Beobachtungsoptik auch eine Beleuchtungsoptik aufnehmen. Es ist grundsätzlich auch vorstellbar, für die Beleuchtungsoptik zumindest einen separaten Kanal vorzusehen. Es ist auch vorstellbar, Kanäle für die Zufuhr und/oder Abfuhr von Fluiden (etwa Insufflationsgas, Spülflüssigkeiten oder dergleichen) zu nutzen. Die Unterteilung solcher sondenartiger Instrumente in mehrere Kanäle vereinfacht den Betrieb und die Handhabung, da sich beispielsweise die Reinigung erleichtert. Ferner ist beispielsweise die Beobachtungsoptik in dem dieser zugeordneten Kanal hinreichend mechanisch geschützt, so dass Manipulationen/Bewegungen in demjenigen Kanal, der für die Durchführung chirurgischer Instrumente vorgesehen ist, keine nachteiligen Auswirkungen auf die Beleuchtungsoptik haben.

Zumindest in beispielhaften Ausführungen ist derjenige Teil des Instruments, welcher tatsächlich in das Körperinnere eingeführt wird, mit einem runden, ovalen oder gar kreisförmigen Querschnitt versehen. Dies ist nicht einschränkend zu verstehen, gleichwohl wird üblicherweise eine möglichst verträgliche äußere Form angestrebt, um ein etwaiges Trauma für den Patienten oder allgemein die Belastung bei der medizinischen Prozedur zu minimieren. Daher besteht ein Ziel darin, den Durchmesser bzw. die Querschnittsfläche eines solchen Sondenteils insgesamt möglichst klein zu gestalten

Weitere Herausforderungen ergeben sich durch die gewünschte Bereitstellung von Bildsignalen. Dies umfasst in beispielhaften Ausführungen die Bereitstellung einer Beobachtungsoptik, welche mit einer Bildaufnahmeeinheit (Bildsensor) gekoppelt ist, so dass eine unmittelbare oder mittelbare Wiedergabe des Sichtfeldes (im Körper) ermöglicht ist. Dies umfasst in beispielhaften Ausführungsformen eine distale Anordnung der Beobachtungsoptik sowie der Bildaufnahmeeinheit (distal am Sondenteil). Die Bildaufnahmeeinheit umfasst einen oder mehrere Bildsensoren sowie weitere elektronische Bauelemente. Die Bildaufnahmeeinheit stellt ein elektronisches (digitales) Bildsignal bereit, welches am proximalen Ende des Sondenteils abgegriffen wird. Die Beobachtungsoptik wird regelmäßig um eine Beleuchtungseinheit ergänzt.

Die Beleuchtungseinheit und die Bildaufnahmeeinheit bedingen regelmäßig einen bestimmten Wärmeeintrag in das Instrument und mittelbar in den Patienten. Auch hier gilt es, übermäßige Belastungen zu vermeiden. Demgemäß muss überschüssige Wärme abgeführt werden.

Im Rahmen der vorliegenden Offenbarung bezieht sich der Begriff distal auf denjenigen Abschnitt/Bereich des Instruments, der vom Benutzer abgewandt ist. Mit anderen Worten ist ein distales Ende des Instruments während der medizinischen Prozedur regelmäßig in das Körperinnere eingeführt, zumindest bei endoskopischen Instrumenten. Im Rahmen der vorliegenden Offenbarung bezieht sich der Begriff proximal auf einen vom distalen Ende abgewandten Abschnitt/Bereich des Instruments, der dem Benutzer zugewandt ist. Somit umfasst ein Instrument mit einem schaftförmig gestalteten Sondenteil beispielsweise einen Rohrkörper, der sich zwischen einem distalen und einem proximalen Ende erstreckt. Dies kann Gestaltungen umfassen, bei denen während der medizinischen Prozedur das distale Ende im Körper und das proximale Ende außerhalb des Körpers angeordnet ist. Dies ist jedoch nicht einschränkend zu verstehen.

Die Herstellung von Schäften/Rohrkörpern für Sondenteile chirurgischer Instrumente ist aufgrund verschiedener Randbedingungen häufig mit großem Aufwand verbunden, vor allem wenn mehrere Kanäle vorgesehen sind. Einerseits ergeben sich Herausforderungen durch die Miniaturisierung. Ferner ist regelmäßig eine gute und einfache Reinigung bzw. Aufbereitung (Sterilisation, etc.) gefordert. Darüber hinaus sollen sich die verwendeten Materialien für medizinische Anwendungen eignen.

Vor diesem Hintergrund liegt der vorliegenden Erfindung die Aufgabe zugrunde, ein medizinisches Instrument zur Bereitstellung eines Zugangs zum Körperinneren mit einem Schaft mit zumindest einem ersten Kanal und einem zweiten Kanal sowie ein geeignetes Verfahren zu dessen Herstellung anzugeben. Das Instrument soll die erforderlichen Funktionen bereitstellen und konstruktiv an das Funktionsspektrum angepasst sein. Dies umfasst auch eine gute Handhabbarkeit.

Die Fertigung soll in einem hierfür geeigneten Verfahren erfolgen, so dass bei gegebenem Querschnitt des Schaftes zumindest zwei Kanäle vorgesehen sind und gegebenenfalls weitere Funktionen integriert werden können. Die Herstellung soll mit möglichst wenigen Schritten erfolgen und eine an den Einsatzzweck angepasste Gestaltung ermöglichen, insbesondere betreffend auch die Reinigung und Aufbereitung des Instruments. Ferner soll der Aufwand für die Herstellung des Instruments verringert werden, insbesondere auch betreffend manuellen Zusatzaufwand. Darüber hinaus soll das Instrument wiederholgenau und prozesssicher herstellbar sein. Zumindest in beispielhaften Ausgestaltungen soll das Instrument die Implementierung möglichst leistungsfähiger Bildaufnehmer ermöglichen, Abwärme solcher Bauelemente soll effizient abgeführt werden.

Gemäß einem ersten Aspekt wird die der Offenbarung zugrunde liegende Aufgabe durch ein medizinisches Instrument zur Bereitstellung eines Zugangs zum Körperinneren gelöst, insbesondere einen Spatel für ein endoskopisches Instrument, das Folgendes aufweist:
- einen Schaft mit einem langgestreckten Grundkörper, wobei sich der Grundkörper zwischen einem distalen Ende und einem proximalen Ende des Schaftes erstreckt,
- einen mit dem Grundkörper des Schaftes an dessen proximalem Ende gekoppelten Griff,
   wobei der Schaft zumindest einen ersten Kanal und einen zweiten Kanal definiert, die sich durch den Schaft erstrecken,
   wobei der erste Kanal eine Passage für chirurgische Instrumente durch den Schaft hindurch bereitstellt,
   wobei der zweite Kanal zur Aufnahme einer Beobachtungsoptik ausgebildet ist, und
   wobei dem zweiten Kanal zumindest ein Nebenkanal benachbart ist, der mit dem zweiten Kanal verbunden ist.

Die Aufgabe der Offenbarung wird auf diese Weise vollkommen gelöst.

Erfindungsgemäß erlaubt nämlich die Gestaltung des Bauteils eine effiziente Wärmeabfuhr über den zumindest einen Nebenkanal. Dies ermöglicht die Verwendung leistungsfähiger Bildaufnehmer und Beleuchtungseinheiten, die entsprechende Verlustwärme erzeugen.

Der Grundkörper ist insbesondere als geschlitztes Rohr gestaltet, vorzugsweise als durchgehend geschlitztes Rohr. Auf diese Weise können zusätzliche Instrumente in einfacher Weise in den ersten Kanal eingebracht werden.

Es versteht sich, dass Begriffe wie erster Kanal und zweiter Kanal nicht als qualitative Abgrenzung zu verstehen sind. Die genutzte Nummerierung dient primär zu Unterscheidungszwecken. Der erste Kanal kann auch als Instrumentenkanal bzw. Instrumentenpassage bezeichnet werden. Der zweite Kanal kann auch als Beobachtungskanal bezeichnet werden. Der zumindest eine Nebenkanal kann auch als Kühlkanal bezeichnet werden.

Der Nebenkanal kann parallel zum zweiten Kanal ausgebildet sein. Dies ist jedoch keine notwendige Voraussetzung. Beispielweise dann, wenn der den Schaft bildende Grundkörper verjüngt ist und Achsen der (gegebenenfalls außermittig angeordneten) Kanäle dieser Verjüngung folgen, kann es benachbarte, aber nicht streng parallele Anordnungen der Kanäle geben.

Zumindest in einer beispielhaften Ausgestaltung ist der zweite Kanal innerhalb des ersten Kanals vorgesehen. Ferner ist es auch vorstellbar, dass der zumindest eine Nebenkanal innerhalb des ersten Kanals ausgebildet ist.

Gemäß einer beispielhaften Ausgestaltung ist der Grundkörper des Schaftes integral gestaltet, wobei der Grundkörper vorzugsweise durch ein additives Fertigungsverfahren erzeugt ist. Mit anderen Worten kann der Grundkörper einstückig gestaltet sein. Vorzugsweise wird der gesamte Schaft durch den Grundkörper gebildet.

Insgesamt ist, zumindest in beispielhaften Ausgestaltungen, eine Konfiguration des Instruments denkbar, die den Schaft und einen Griff zur Handhabung des Schaftes umfasst, wobei gegebenenfalls ein Verbindungsstück zwischen dem Schaft und dem Griff vorgesehen ist. Der Griff kann beispielhaft als Handgriff gestaltet sein. Der Griff kann grundsätzlich auch Teil eines Gehäuses am proximalen Ende des Schaftes sein. Der spaltelförmige Schaft wird über den Griff geführt.

Die additive Fertigung erlaubt die Erzeugung mehrerer Kanäle in einem einstückig gestalteten Bauteil, wobei die Kanäle bedarfsweise miteinander gekoppelt sein können. Insbesondere derartige Verbindungen lassen sich mit konventionellen Herstellverfahren bei einstückigen Bauteilen nicht oder nur mit hohem Aufwand herstellen.

Da die Möglichkeit besteht, zusätzliche Nebenkanäle in den Schaft des Instruments einzubringen, lässt sich beispielsweise die Wärmeabfuhr verbessern. Somit besteht grundsätzlich die Möglichkeit, leistungsfähigere Beobachtungsoptiken, Beleuchtungseinheiten und (elektronische) Bildaufnehmer zu verbauen. Da nunmehr die Wärme effizient abgeführt werden kann, erhöht sich die Belastung für den Patienten nicht.

Die Gestaltung des Schaftes bzw. von dessen Grundkörper lässt sich an die anatomischen Gegebenheiten anpassen. Insbesondere können ineinander liegende Kanäle realisiert werden, wobei Engstellen, Innenkanten, Auskehlungen und ähnliche problematische Gestaltelemente vermeidbar sind. Oberflächen können insgesamt rund und mit geglätteten Übergängen gestaltet werden. Vorteile ergeben sich beispielsweise bei der Reinigung bzw. Aufbereitung des Instruments. Je besser die Elemente bei der Reinigung erreichbar sind, umso geringer ist der Aufwand.

Die integrale, einstückige Gestaltung, umfassend auch die Ausbildung der Kanäle, sorgt ferner dafür, dass sich der Aufwand für die Bearbeitung - insbesondere hinsichtlich der einzelnen Bearbeitungsschritte - reduziert. Im Idealfall ist keine umfassende mechanische abtragende Bearbeitung nötig. Dies ist nicht einschränkend zu verstehen. Beispielsweise kann im Einzelfall sehr wohl eine abtragende Nachbearbeitung zur Verbesserung von Oberflächeneigenschaften vorgesehen sein. Auch wenn der Schritt der additiven Herstellung eine gewisse Zeit in Anspruch nimmt, so lässt sich insgesamt eine Reduktion der Herstellungszeit erreichen.

Ein weiterer Vorteil besteht darin, dass der Aufwand für spezifische Betriebsmittel für die Herstellung des Schaftes sinkt. Im Idealfall kann die gesamte Gestalt des Grundkörpers des Schaftes in nur einem Arbeitsgang erzeugt werden, gegebenenfalls ergänzt um wenige Nachbearbeitungsmaßnahmen.

Die einstückige Gestaltung hat den weiteren Vorteil, dass der gesamte Schaft bzw. dessen Grundkörper aus ein und demselben Werkstoff herstellbar ist. Insbesondere können Werkstoffe mit günstigen Eigenschaften hinsichtlich der Biokompatibilität und/oder Patientenverträglichkeit gewählt werden. Beispielsweise bieten sich austenitische, rostfreie Stahlwerkstoffe an. Die additive Fertigung hat ferner Vorteile hinsichtlich des tatsächlichen Werkstoffverbrauchs.

Der zu erzeugende Grundkörper kann gezielt an zu erwartende Belastungen angepasst werden. Es gibt nur wenige Einschränkungen bei der Gestaltung. Das zu erzeugende Bauteil kann gezielt an potenziell hochbelasteten Stellen verstärkt werden. Minderbelastete Stellen können dünner/leichter ausfallen.

Die additive Fertigung ermöglicht bei einstückig gestalteten Bauteilen die Integration von Durchgangslöchern, Sacklöchern und insbesondere dazwischen vorgesehene Verbindungen. Sowohl Sacklöcher als auch Querverbindungen zwischen einzelnen Kanälen lassen sich mit konventionellen Fertigungsansätzen bei einstückig gestalteten Bauteilen nur mit hohem Aufwand verwirklichen, wenn überhaupt.

Gemäß einer weiteren beispielhaften Ausgestaltung des Instruments ist der Grundkörper zumindest abschnittsweise geschlitzt. Vorzugsweise ist der Grundkörper vollständig entlang seiner Längserstreckung zwischen dem distalen und dem proximalen Ende geschlitzt. Die geschlitzte Gestaltung des Grundkörpers ist ohne subtraktive Maßnahmen möglich. Der entsprechende Raum wird bei der additiven Fertigung lediglich bei der Verbindung/Vernetzung der Partikel ausgespart.

Ferner besteht die Möglichkeit, bestimmte Abschnitte des Bauteils zu verstärken, so dass trotz durchgehender Gestaltung des Schlitzes eine hinreichende konstruktive Stabilität gegeben ist. Der Schlitz erlaubt eine seitliche Zugänglichkeit des ersten Kanals, insbesondere eine radiale Zugänglichkeit. Auf diese Weise können weitere Instrumente besonders einfach seitlich in den ersten Kanal eingebracht werden.

Gemäß einer weiteren beispielhaften Ausgestaltung des Instruments beherbergt der zweite Kanal neben der Beobachtungsoptik ferner eine Beleuchtungseinheit. Die Beleuchtungseinheit umfasst beispielsweise einen Lichtleiter, der sich vom proximalen Ende des Schaftes hin zum distalen Ende erstreckt. Es ist grundsätzlich auch vorstellbar, alternativ zum Lichtleiter oder in Ergänzung dazu, zumindest eine LED o. ä. Lichtquellen direkt im zweiten Kanal vorzusehen. Die Beleuchtungseinheit kann grundsätzlich auch eine Beleuchtungsoptik umfassen.

Mit anderen Worten teilen sich die Beobachtungsoptik und die Beleuchtungseinheit den zur Verfügung stehenden Raum im zweiten Kanal. Es ist vorstellbar, den zweiten Kanal durch darin eingebrachte (Innen-)Schäfte in der gewünschten Weise zu unterteilen. Die Innenschäfte stellen jeweils eine Aufnahme für die Beobachtungsoptik und/oder die Beleuchtungseinheit sowie gegebenenfalls eine Abgrenzung dazwischen bereit.

Gemäß einer weiteren beispielhaften Ausgestaltung des Instruments ist der zweite Kanal über einen Verbindungskanal mit dem zumindest einen Nebenkanal verbunden. Es versteht sich, dass auch mehrere Verbindungskanäle vorstellbar sind.

Ein solcher Verbindungskanal ist beispielsweise als Querverbindung (im Wesentlichen quer zur Längserstreckung des Schaftes) gestaltet. Die Erzeugung des Verbindungskanals ist mit konventionellen Fertigungsmethoden nicht oder nur mit hohem Aufwand möglich.

Der zumindest eine Verbindungskanal kann zur Verbesserung der Wärmeabfuhr genutzt werden. Beispielsweise kann der zumindest eine Verbindungskanal an einen besonders wärmebelasteten Bereich des zweiten Kanals ankoppeln. Beispielhaft ist der zumindest eine Verbindungskanal dem distalen Ende des Grundkörpers benachbart bzw. in einem distalen Endbereich des Grundkörpers angeordnet. Der zumindest eine der zumindest eine Verbindungskanal ist ferner beispielsweise mit einem besonders gut wärmenabführenden Stoff gefüllt (Stoff mit guter Wärmeleitfähigkeit). Auf diese Weise kann Wärme gezielt vom zweiten Kanal in Richtung auf den zumindest einen Nebenkanal abgeführt werden. Somit muss die Abwärme nicht oder nicht vollständig über den zweiten Kanal selbst abgeführt werden.

Auf diese Weise können beim distalen Ende des Grundkörpers Bauteile mit hoher Verlustleistung angeordnet werden. Es kann sich dabei insbesondere um besonders leistungsfähige Bildaufnehmer mit entsprechender Elektronik handeln, welche von der Beobachtungsoptik bereitgestellte Bilder erfassen und in digitale Informationen überführen.

Gemäß einer weiteren beispielhaften Ausgestaltung des Instruments ist der Verbindungskanal in einem distalen Endbereich oder benachbart zu diesem angeordnet. Generell ist es bevorzugt, wenn der Verbindungskanal in der Nähe von wärmeerzeugenden bzw. wärmeabgebenden Bauteilen im zweiten Kanal angeordnet ist.

Gemäß einer weiteren beispielhaften Ausgestaltung des Instruments ist der zumindest eine Nebenkanal als Sackloch (Blindloch) gestaltet und am distalen Ende des Schaftes geschlossen. Dies hat den Vorteil, dass der zumindest eine Nebenkanal sicher in Richtung auf das distale Ende des Schaftes abgedichtet ist. Somit wird ein Übertritt von Medien über den zumindest einen Nebenkanal erschwert. Die Gestaltung als Sackloch umfasst vorzugsweise eine Ausbildung ohne Senkung. Mit anderen Worten kann das Sackloch zumindest in beispielhaften Ausgestaltungen einen ebenen Boden in Richtung auf das distale Ende des Schaftes aufweisen.

Gemäß einer weiteren beispielhaften Ausgestaltung des Instruments beherbergt der zumindest eine Nebenkanal ein Wärmeabfuhrelement, um Wärme vom distalen Ende hin zum proximalen Ende abzuführen. Allgemein handelt sich dabei beispielsweise um ein Element aus einem Werkstoff mit guter Wärmeleitfähigkeit. Es ist grundsätzlich auch vorstellbar, ein Wärmeabfuhrelement vorzusehen, welches durchströmbar ist.

Gemäß einer weiteren beispielhaften Ausgestaltung des Instruments ist der zweite Kanal zwischen zwei Nebenkanälen angeordnet, die einen distalen Endbereich des Kanals mit dem proximalen Ende des Instruments verbinden.

Bei einer beispielhaften Gestaltung, bei der der zweite Kanal im ersten Kanal ausgebildet ist, sind die beiden Nebenkanäle beidseits des zweiten Kanals angeordnet, so dass insgesamt der für den ersten Kanal bereitstehende Bauraum (für den dort offenen Querschnitt für die Passage) nicht übermäßig reduziert wird. Mit anderen Worten können die beiden Nebenkanäle in dem Bereich angeordnet sein, in dem bei konventioneller Fertigung mit zwei ineinander angeordneten Rohrkörpern eine Auskehlung/Innenkante zwischen dem Rohrkörper des zweiten Kanals und der Umfangswand des ersten Kanals gegeben ist. Somit gibt es für den Bediener bei gegebener Außenabmessung des Grundkörpers keine wesentlichen Einschränkungen bei der im ersten Kanal für Instrumente und Ähnliches vorgesehenen Passage. Es ist grundsätzlich auch vorstellbar, weitere Nebenkanäle vorzusehen. Die additive Fertigung ermöglicht hier große Gestaltungsfreiheit.

Gemäß einer weiteren beispielhaften Ausgestaltung des Instruments weist der erste Kanal einen nierenförmigen Querschnitt auf. Dies trifft insbesondere auf die im ersten Kanal gebildete Passage zu. Der zweite Kanal ist gemäß dieser Ausgestaltung einem konkaven Abschnitt des nierenförmigen Querschnitts benachbart. Der nierenförmige Querschnitt weist konvexe (nach außen gekrümmte) Abschnitte und an einer Seite einen konkaven (nach innen gekrümmten) Abschnitt auf. Diese Definition bezieht sich auf die bereitgestellte Passage/Ausnehmung. Bei umgekehrter Definition unter Bezug auf die Wand kann auch eine umgekehrte Zuordnung der konvexen/konkaven Abschnitte gewählt werden.

Der nierenförmige Querschnitt ähnelt einem Oval oder Langloch mit einer kurzen Hauptachse und einer langen Hauptachse, wobei entlang der langen Hauptachse eine der beiden äußeren Seiten mit dem konkaven (nach innen gekrümmten) Abschnitt versehen ist. Vorzugsweise weist der Querschnitt runde, insbesondere tangentiale Übergänge auf. Auf diese Weise können Kanten, Ecken, Engstellen und dergleichen vermieden werden.

Gemäß einer weiteren beispielhaften Ausgestaltung ist der zweite Kanal mit einem kreisförmigen Querschnitt versehen und in ein gedachtes Oval des ersten Kanals eingerückt, um dort den nierenförmigen Querschnitt zu bilden.

Gemäß einer weiteren beispielhaften Ausgestaltung des Instruments ist der zweite Kanal, in einer Querschnittsrichtung des Schaftes betrachtet, insbesondere bei Betrachtung einer gedachten Längsmittelebene durch den Schaft, zwischen dem ersten Kanal und dem Griff angeordnet. Ferner ist der zumindest eine Nebenkanal in einer weiteren beispielhaften Ausgestaltung seitlich der gedachten Längsmittelebene durch den Schaft angeordnet. Dies trifft vorzugweise auf beide Nebenkanäle zu. Die gedachte Längsmittelebene wird durch ein Zentrum (beispielsweise Schnittpunkt der beiden Hauptachsen) des ersten Kanals und ein Zentrum des zweiten Kanals gebildet. Beispielhaft bildet die Längsmittelebene eine Symmetrieachse des Schaftes. Beispielhaft bildet die Längsmittelebene ferner eine Symmetrieachse des Griffes. Der seitliche Schlitz im Schaft ist von der gedachten Längsmittelebene versetzt, insbesondere in einem hiervon abgewandten Bereich des Mantels (Umfangswand) des ersten Kanals vorgesehen.

Gemäß einer weiteren beispielhaften Ausgestaltung des Instruments weist der Grundkörper des Schaftes an seinem Umfangsbereich, der dem Griff (Handgriff) zugewandt ist, eine Materialanhäufung auf, welche den zweiten Kanal und die zumindest einen Nebenkanal beherbergt. Die additive Fertigung ermöglicht in weiten Grenzen beliebige Wandstärken bei der Ausbildung des Grundkörpers. Auf diese Weise kann die Materialanhäufung ohne großen Zusatzaufwand gebildet werden. Demgemäß steht genügend Material zur Verfügung, um dort den zweiten Kanal und den zumindest einen Nebenkanal auszubilden. Ferner weist die Materialanhäufung ein günstiges Widerstandsmoment (gegen Biegung und dergleichen) auf, so dass sich insgesamt die Stabilität des Grundkörpers und des Schaftes erhöht. Auf diese Weise kann etwa der seitliche Schlitz durchgehend gestaltet werden, so dass sich insgesamt für den ersten Kanal ein offenes Profil ergibt. Gleichwohl weist der Schaft eine hinreichend hohe Festigkeit auf.

Gemäß einer weiteren beispielhaften Ausgestaltung des Instruments verjüngt sich der Grundkörper ausgehend vom proximalen Ende in Richtung des distalen Endes zumindest abschnittsweise, wobei sich der Grundkörper vorzugsweise kontinuierlich verjüngt. Auf diese Weise kann der Schaft insgesamt besser eingeführt werden. Auch hier erlaubt die additive Fertigung in weiten Grenzen eine anwendungsspezifische Gestaltung.

Gemäß einer weiteren beispielhaften Ausgestaltung des Instruments ist am distalen Ende des Schaftes beim Grundkörper eine Spitze ausgebildet, die insbesondere durch einen schrägen Abschnitt des Grundkörpers gebildet ist. Somit ist die Spitze etwa zungenartig oder muldenartig gestaltet. Auf diese Weise kann das Instrument ohne großen Zusatzaufwand durch die additive Fertigung günstig an die Einsatzbedingungen angepasst werden.

Gemäß einer weiteren beispielhaften Ausgestaltung des Instruments beherbergt der zweite Kanal in seinem distalen Endbereich eine Objektivbaugruppe, die einem Bildaufnehmer vorgelagert ist. Der zumindest eine Nebenkanal erlaubt eine effiziente Wärmeabfuhr, auch von im distalen Bereich angeordneten Bauelementen, so dass die distale Anordnung leistungsfähiger Bildaufnehmer und Objektivbaugruppen sowie entsprechender Elemente zur Beleuchtung des Sichtfeldes möglich wird.

Gemäß einer weiteren beispielhaften Ausgestaltung des Instruments ist der zumindest eine Nebenkanal zur Abfuhr von Abwärme des Bildaufnehmers oder der Objektivbaugruppe ausgebildet und vorzugsweise über den Verbindungskanal seitlich an den distalen Endbereich angekoppelt.

Der zumindest eine Bildaufnehmer ist beispielhaft Bestandteil einer Bildaufnahmeeinheit, welche einen oder mehrere Bildaufnehmer umfasst. Auf diese Weise können ein Beobachtungskanal oder sogar zwei Beobachtungskanäle (Stereo-Beobachtung) realisiert werden. Die Bildaufnehmer können beispielhaft durch CCD-Sensoren gebildet sein. Ferner können Steuerschaltungen und ähnliche Bauelemente direkt beim distalen Ende des Schaftes angeordnet sein.

Gemäß einem weiteren Aspekt wird die der Offenbarung zugrunde liegende Aufgabe durch ein Verfahren zur Herstellung eines medizinischen Instruments zur Bereitstellung eines Zugangs zum Körperinneren gelöst, insbesondere eines Spatels für ein endoskopisches Instrument, wobei das Verfahren die folgenden Schritte aufweist:
- Bereitstellung einer Datenverkörperung eines Schaftes mit einem langgestreckten Grundkörper,
   wobei sich der Grundkörper zwischen einem distalen Ende und einem proximalen Ende des Schaftes erstreckt,
   wobei der Schaft zumindest einen ersten Kanal und einen zweiten Kanal definiert, die sich durch den Schaft erstrecken,
   wobei der erste Kanal eine Passage für chirurgische Instrumente durch den Schaft hindurch bereitstellt,
   wobei der zweite Kanal zur Aufnahme einer Beobachtungsoptik ausgestaltet ist, und
   wobei dem zweiten Kanal zumindest ein Nebenkanal benachbart ist, der mit dem zweiten Kanal verbunden ist,
- integrale Fertigung des Schaftes mit dem ersten Kanal, dem zweiten Kanal und dem zumindest einen Nebenkanal in einem additiven Fertigungsverfahren auf Basis eines pulverförmigen metallischen Ausgangsmaterials unter Berücksichtigung der Datenverkörperung, und
- Verbindung des Schaftes mit einem Griff am proximalen Ende des Grundkörpers des Schaftes.

Auch auf diese Weise wird die Aufgabe der Offenbarung vollkommen gelöst.

Das Verfahren eignet sich insbesondere zur Herstellung eines Bauteils gemäß einer der zuvor beschriebenen Ausgestaltungen. Die additive Fertigung ermöglicht hohe Gestaltungsfreiheit, so dass der Grundkörper, insbesondere der gesamte Schaft, vollständig oder weitgehend integral/einstückig gestaltet werden können. Trotz integraler Gestaltung lassen sich diverse Kanäle in den Grundkörper einbringen, ferner ist die Erzeugung von Querverbindungen zwischen den Kanälen möglich. Dies ist auch bei Kanälen vorgesehen, die sich nicht vollständig (als Durchgangslöcher) durch den Grundkörper erstrecken. Solche den Grundkörper nicht vollständig durchdringende Kanäle (Sacklöcher) können durch die additive Fertigung auch in unmittelbarer Nachbarschaft des distalen Endes, an dem die Kanäle blind enden, mit weiteren Kanälen verbunden werden. Dies ist mit konventionellen Fertigungsverfahren nicht oder nur mit übermäßig hohem Aufwand möglich.

Auch die Erzeugung geschlitzter Rohre und ineinander angeordneter/verschachtelter Kanäle ist durch die additive Fertigung einfach möglich. Ferner erlaubt die additive Fertigung glatte Übergänge zwischen verschiedenen Gestaltelementen, wodurch die Reinigung/Aufbereitung weiter vereinfacht wird.

Gemäß einer beispielhaften Ausgestaltung des Verfahrens umfasst der Schritt der additiven Fertigung des Schaftes eine Fertigung auf Basis eines pulverförmigen austenitischen, rostfreien Stahlwerkstoffs. Ein derartiger Werkstoff ist für medizinische Anwendungen geeignet. Es gibt also eine gute Verträglichkeit für den Patienten. Ferner eignen sich solche Werkstoffe für die Reinigung/Aufbereitung zwischen verschiedenen Anwendungen. Beispielhaft kann es sich bei dem Werkstoff um einen Edelstahl mit der Werkstoffnummer 1.4404 handeln. Solche Edelstähle weisen eine hohe Korrosionsbeständigkeit auf.

Gemäß einer weiteren beispielhaften Ausgestaltung des Verfahrens umfasst der Schritt der Fertigung die Nutzung einer Pulverbett basierten Vorrichtung zur additiven Fertigung, wobei Pulver in einem Bauraum der Vorrichtung durch energiereiche Strahlung erweicht und gefügt wird. Beispielhaft kann es sich um ein SLM-Verfahren (Selektives Laserschmelzen) handeln. Vorstellbar ist grundsätzlich auch ein SLS-Verfahren (Selektives Lasersintern). Beides sind sog. Pulverbett-Verfahren.

Bei dem SLM-Verfahren liegt der Werkstoff pulverförmig vor, wobei aus einer Vorratskammer Material in eine Baukammer übertragen und dort schichtweise abgelegt wird, wobei in der Baukammer eine jeweils um die Dicke einer Schicht absenkbare Plattform vorgesehen ist. Die jeweils oberste Schicht wird mit einem Laserstrahl zumindest teilweise geschmolzen, so dass Schicht für Schicht insgesamt ein festes Bauteil mit der gewünschten Geometrie erzeugt wird. Bereiche, in denen das Pulver nicht geschmolzen wird, werden nach dem Aufbau gereinigt, so dass im Ergebnis das weitgehend oder vollständig fertige Bauteil mit den gewünschten Kavitäten/Kanälen vorliegt.

Gemäß einer weiteren beispielhaften Ausgestaltung des Verfahrens erfolgt die Fertigung des Grundkörpers aufrecht stehend, mit vertikaler Orientierung einer Längsachse in Bezug auf die Bauplattform. Querschnittsbereiche ("Scheiben") des Grundkörpers werden gleichzeitig oder zumindest zeitlich überlappend gefertigt. Auf diese Weise ergeben sich hinreichend homogene Eigenschaften im jeweiligen Querschnittsbereich.

Gemäß einer weiteren beispielhaften Ausgestaltung des Verfahrens umfasst der Schritt der Bereitstellung der Datenverkörperung eine Bereitstellung einer einen erwarteten Verzug des Bauteils bei der Fertigung vorwegnehmenden Datenverkörperung. Mit anderen Worten kann aufgrund von Erfahrungen und/oder Simulationen der Verzug des Bauteils vorhergesagt werden, so dass der Verzug "vorgehalten" werden kann. Wenn nun ein künstlich in entgegengesetzter Richtung verzogenes Bauteil der Fertigung zugrunde gelegt wird und sich der erwartete Verzug einstellt, so ergibt sich im Ergebnis die gewünschte Gestalt.

Eine Datenverkörperung ist ein digitales Abbild der Gestalt des Bauteils. Das digitale Abbild kann um weitere Fertigungsinformationen ergänzt sein. Gemäß einer beispielhaften Ausgestaltung des Verfahrens wird bei der Bereitstellung der den erwarteten Verzug vorwegnehmenden Datenverkörperung ein sich aufgrund inhomogener Materialverteilung bzw. Materialanhäufungen ergebender Verzug berücksichtigt.

Gemäß einer weiteren beispielhaften Ausgestaltung umfasst das Verfahren ferner zumindest einen abtragenden Nachbearbeitungsschritt, der ein Strömungsschleifen zumindest des zweiten Kanals umfasst. Beim Strömungsschleifen wird Schleifmittel durch eine Fluidströmung angetrieben und entlang des Werkstücks bewegt. Auf diese Weise kann beispielsweise im zweiten Kanal die gewünschte Oberflächenqualität erzeugt werden. Grundsätzlich ist auch ein derartiges Verfahren beim ersten Kanal vorstellbar.

In beispielhaften Ausgestaltungen wird bei dem zumindest einen Nebenkanal auf ein Strömungsschleifen verzichtet, da der Nebenkanal nicht als Durchgangsloch gestaltet ist. Wenn kein Übertritt von Substanzen durch den Nebenkanal in Richtung auf das Körperinnere droht, sind mit dem additiven Verfahren erzielbare Oberflächenqualitäten ausreichend.

Es versteht sich, dass die vorstehend genannten und die nachstehend noch zu erläuternden Merkmale der Erfindung nicht nur in der jeweils angegebenen Kombination, sondern auch in anderen Kombinationen oder in Alleinstellung verwendbar sind, ohne den Rahmen der vorliegenden Erfindung zu verlassen.

Weitere Merkmale und Vorteile der Erfindung ergeben sich aus der nachfolgenden Beschreibung mehrerer Ausführungsbeispiele unter Bezugnahme auf die Zeichnungen. Es zeigen:
- Fig. 1:: eine perspektivische rückwärtige Ansicht einer Ausgestaltung eines medizinischen Instruments in Form eines Video-Mediastinoskops;
- Fig. 2:: eine geschnittene Ansicht durch den Schaft des Instruments gemäß Fig. 1;
- Fig. 3:: eine teilweise explodierte perspektivische Ansicht einer Ausgestaltung eines medizinischen Instruments in Form eines Video-Mediastinoskops, wobei aus Veranschaulichungszwecken ein Schaft separat dargestellt ist;
- Fig. 4:: eine vergrößerte Teilansicht der Ausgestaltung gemäß Fig. 3, zur Veranschaulichung einer Objektivbaugruppe des Instruments;
- Fig. 5:: eine seitliche Ansicht des Instrumentenschaftes der Ausgestaltung gemäß Fig. 3;
- Fig. 6:: eine rückwärtige Ansicht des Instrumentenschaftes gemäß Fig. 5;
- Fig. 7:: einen Schnitt durch den Instrumentenschaft gemäß Fig. 5 entlang der Linie VII-VII in Fig. 5;
- Fig. 8:: einen Längsschnitt durch den Instrumentenschaft gemäß Fig. 5 entlang der Linie VIII-VIII in Fig. 6;
- Fig. 9:: eine vergrößerte Teilansicht des distalen Endes des Instrumentenschaftes gemäß Fig. 8;
- Fig. 10:: eine Seitenansicht einer Ausgestaltung eines Instrumentenschaftes mit fertigungsbedingtem Verzug;
- Fig. 11:: eine Seitenansicht eines Datenmodells des Instrumentenschaftes gemäß Fig. 10 mit vorweggenommenem Verzug;
- Fig. 12:: eine Seitenansicht eines auf Basis des Datenmodells gemäß Fig. 11 hergestellten Instrumentenschaftes mit fertigungsbedingtem Verzug;
- Fig. 13:: ein Blockdiagramm zur Veranschaulichung einer Ausgestaltung eines Verfahrens zur Herstellung eines medizinischen Instruments; und
- Fig. 14:: ein Blockdiagramm zur Veranschaulichung eines Ansatzes zur Berücksichtigung eines fertigungsbedingten Verzuges bei einem Verfahren zur Herstellung eines medizinischen Instruments.

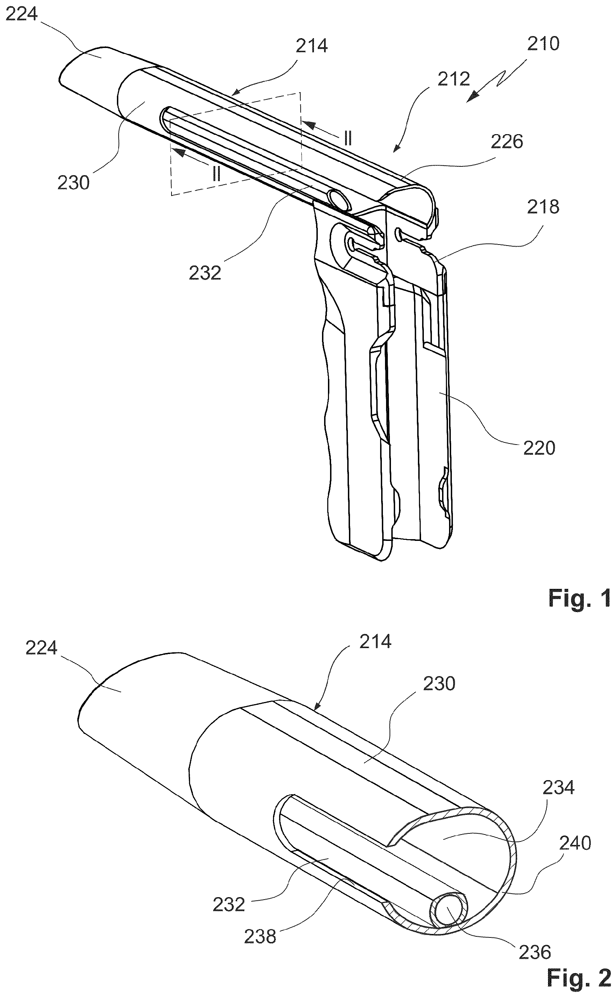

Fig. 1 zeigt anhand einer perspektivischen rückwärtigen Ansicht eine konventionelle Gestaltung eines insgesamt mit 210 bezeichneten Instruments. Beispielhaft handelt es sich bei dem Instrument 210 um ein Mediastinoskop, insbesondere ein Video-Mediastinoskop.

Das Instrument 210 umfasst einen schaftförmig gestalteten Spatel 212. Der Spatel 212 wird durch einen Schaft 214 gebildet. Der Schaft 214 weist eine ausgeprägte Längserstreckung auf. Das Instrument 210 umfasst neben dem Spatel 212 einen Griff 220 sowie ein dazwischen angeordnetes Verbindungsstück 218, welches einen Adapter zwischen dem Spatel 212 und dem Griff 220 bereitstellt. Der den Spatel 212 bildende Schaft 214 erstreckt sich zwischen einem distalen Ende 224 und einem proximalen Ende 226. Im beispielhaften Betrieb als Mediastinoskop wird das distale Ende 224 in das Körperinnere des Patienten eingeführt. Beim proximalen Ende 226 des Schaftes 214 koppelt der Griff 220 über das Verbindungsstück 218 an den Schaft 214 an. Der Griff 220 ist gegenüber der Längserstreckung des Schaftes 214 deutlich geneigt und im Ausführungsbeispiel ähnlich einem Pistolengriff angeordnet.

Fig. 2 veranschaulicht einen Schnitt durch den Schaft 214, wobei die Schnittebene in Fig. 1 durch einen gestrichelten Block II-II angedeutet ist. Der Spatel 212 wird durch ein äußeres Rohr 230 mit gestauchtem Querschnitt bzw. näherungsweise ovalem Querschnitt sowie ein inneres Rohr 232 gebildet. Das innere Rohr 232 weist einen etwa kreisrunden Innenbereich auf. Am Umfang ist das innere Rohr 232 teilweise abgeflacht. Das äußere Rohr 230 und das innere Rohr 232 sind ursprünglich separate Teile, welche miteinander gefügt sind. Die Abflachung des inneren Rohres 232 ist zumindest abschnittsweise an eine Innenkontur des äußeren Rohres 230 angepasst. Damit liegt das innere Rohr 232 vorzugsweise flächig (nicht nur mit Linienberührung) an der Innenwand des äußeren Rohres 230 an. Zwischen den jeweiligen Wandlungen des äußeren Rohres 32 und des inneren Rohrs 132 bilden sich spitze Kehlen, welche den Reinigungsaufwand erhöhen.

Das äußere Rohr 230 (vermindert um den vom inneren Rohr 232 beanspruchten Bauraum) bildet einen ersten Kanal 234. Durch den ersten Kanal 234 können weitere Instrumente in das Körperinnere geführt werden. Auf diese Weise können beispielsweise Biopsien durchgeführt werden. Das innere Rohr 232 bildet einen zweiten Kanal 236. Beispielhaft beherbergt der zweite Kanal 236 eine Objektivbaugruppe, Bildaufnehmer, eine Beleuchtungseinheit, etc. Daher kann der zweite Kanal 236 auch als Beobachtungskanal bezeichnet werden. Demgemäß kann der erste Kanal 234 auch als Instrumentenkanal bezeichnet werden.

Im äußeren Rohr 230 ist ein sich in Längsrichtung erstreckender Schlitz 238 ausgebildet, welcher sich ausgehend vom proximalen Ende 226 in Richtung auf das distale Ende 224 erstreckt. Der Schlitz erstreckt sich jedoch nicht vollständig in der Längsrichtung durch das äußere Rohr 230 bzw. den Schaft 214 hindurch.

Insgesamt sind das äußere Rohr 230 und das innere Rohr 232 ineinander angeordnet, wobei sich der in Fig. 2 veranschaulichte Querschnitt 240 ergibt. Eine solche Querschnittsgestaltung auf Basis zweier Rohrkörper ist mit einer einstückigen konventionellen Herstellung kaum realisierbar, allenfalls mit hohem Aufwand.

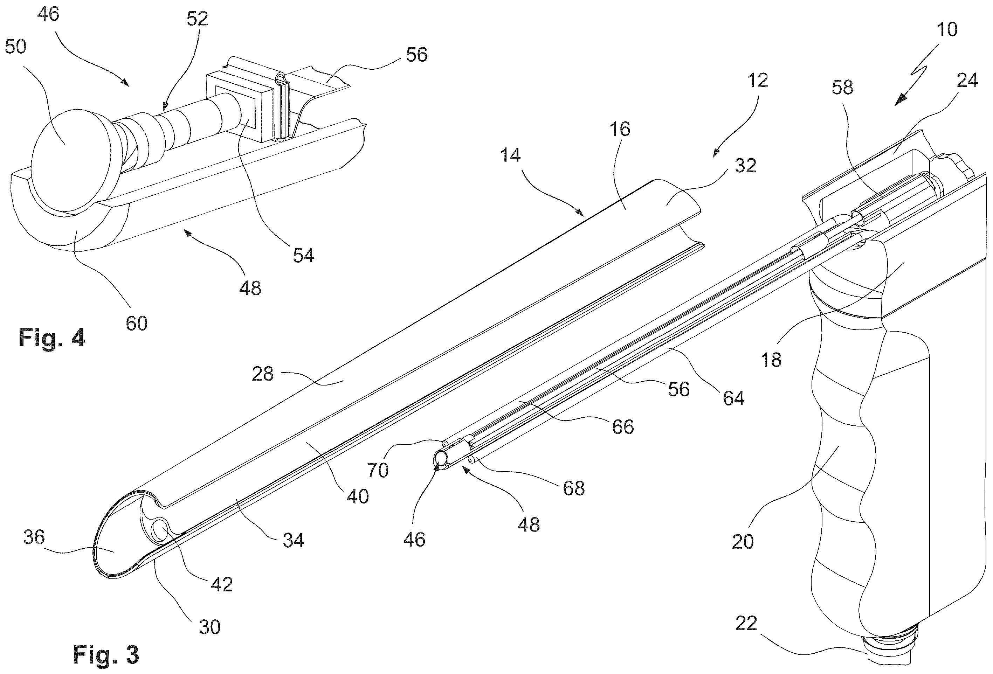

Mit Bezugnahme auf die Figuren 3 bis 12 wird ein Ansatz zur einstückigen Gestaltung von Schäften derartiger und ähnlicher Instrumente zur Bereitstellung eines Zugangs zum Körperinneren veranschaulicht.

Fig. 3 veranschaulicht anhand einer perspektivischen frontalen Ansicht vom distalen Ende her ein insgesamt mit 10 bezeichnetes Instrument. Beispielhaft ist das Instrument 10 als Mediastinoskop, insbesondere als Video-Mediastinoskop ausgebildet. Dies ist jedoch nicht einschränkend zu verstehen. Allgemein kann das Instrument 10 als endoskopisches Instrument gestaltet sein.

Das Instrument 10 weist einen hier als Spatel 12 bezeichneten Schaft 14 auf. Der Schaft 14 wird im Wesentlichen, vorzugweise vollständig durch einen Grundkörper 16 gebildet. Vorzugsweise ist der Grundkörper 16 einstückig (integral) gefertigt. Hierfür eignen sich Verfahren der additiven Fertigung. Der Grundkörper 16 besteht beispielhaft aus einem für medizinische Anwendungen geeigneten Edelstahl.

Der Schaft 14 ist über ein Verbindungsstück 18 mit einem Griff 20 verbunden. Der Griff 20 ist im gezeigten Ausführungsbeispiel als Handgriff gestaltet. Der Griff 20 im Ausführungsbeispiel ist schräg oder quer zum Schaft 14 orientiert, etwa nach Art eines Pistolengriffes. Dies ist nicht einschränkend zu verstehen, der Griff 20 kann auch andersartig gestaltet sein. In Fig. 3 ist ferner mit 22 ein Kabelabgang/Anschluss am Griff 20 angedeutet. Der Anschluss 22 bildet eine Schnittstelle zum Transfer von Energie, Medien, Daten und ähnlichem. Das Verbindungsstück 18 ist beispielhaft mit dem Schaft 14 verschweißt. Es ist grundsätzlich auch vorstellbar, das Verbindungsstück 18 gemeinsam mit dem Schaft 14 einstückig zu gestalten. Am Verbindungsstück 18 ist ein Sitz 24 für den Schaft 14 ausgebildet.

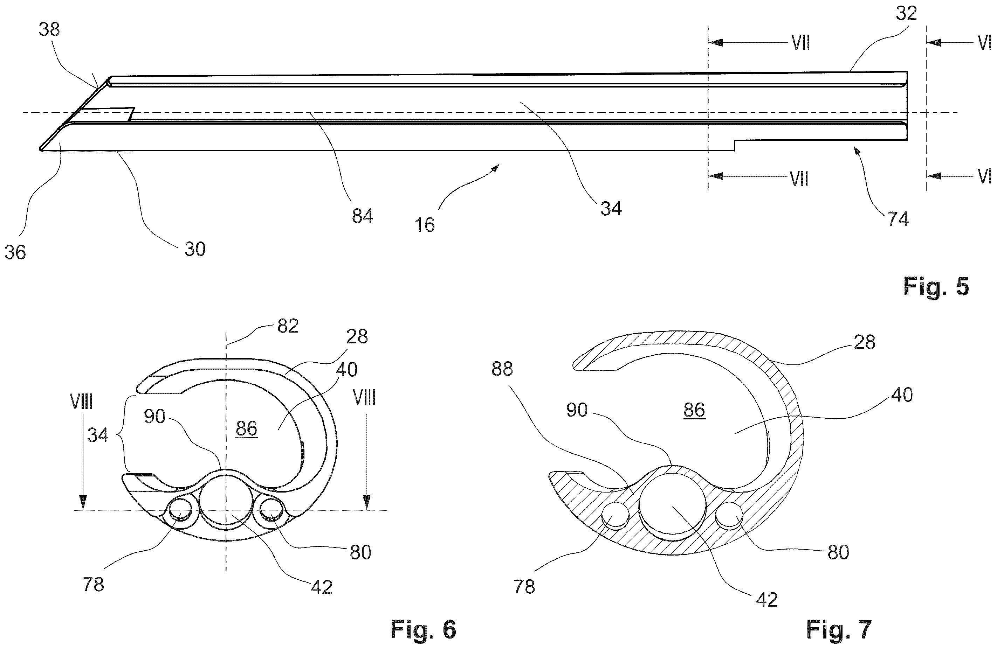

Der Schaft 14 bzw. der den Schaft 14 bildende Grundkörper 16 umfasst einen Mantel 28, der einen Umfangsbereich bzw. eine Umfangswandung definiert. Der Grundkörper 16 erstreckt sich zwischen einem distalen Ende 30 und einem proximalen Ende 32. Die Begriffe distales Ende 30 und proximales Ende 32 beziehen sich jeweils auf einen entsprechenden Abschnitt des Schaftes 14. Zwischen dem distalen Ende 30 und dem proximalen Ende 32 erstreckt sich ein (seitlicher) Schlitz 34. Im gezeigten Ausführungsbeispiel gemäß Fig. 3 erstreckt sich der Schlitz 34 über die gesamte Längserstreckung des Grundkörpers 16. Beim distalen Ende 30 ist eine zungenförmige Spitze 36 ausgebildet. Die Spitze 36 wird durch einen schrägen Abschnitt 38 (vergleiche auch Fig. 5) des Grundkörpers 16 gebildet.

Der Grundkörper 16 bildet einen ersten Kanal 40 und einen zweiten Kanal 42 im Schaft 14 aus. Im Ausführungsbeispiel weist der erste Kanal 40 einen wesentlich größeren Querschnitt als der zweite Kanal 42 auf. Der zweite Kanal 42 ist in einem Randbereich des ersten Kanals 40 ausgebildet. Sowohl der erste Kanal 40 als auch der zweite Kanal 42 erstrecken sich durch den Grundkörper 16 hindurch. Der erste Kanal 40 und der zweite Kanal 42 sind demgemäß als Durchgangslöcher gestaltet. Der erste Kanal 40 bildet eine Passage für Instrumente, um den Zugang zum Körperinneren für diese Instrumente zu vereinfachen.

Der zweite Kanal 42 beherbergt im Ausführungsbeispiel eine Beobachtungsoptik 46 sowie eine Beleuchtungseinheit 48. In diesem Zusammenhang wird auf die vergrößerte Teilansicht gemäß Fig. 4 verwiesen. Fig. 4 veranschaulicht Elemente, die in der gezeigten Ausführungsform beim distalen Ende des zweiten Kanals 42 angeordnet sind. Beispielhaft umfasst die Beobachtungsoptik 46 ein Deckglas 50, welches einen distalen Abschluss bildet, und eine Objektivbaugruppe 52. Die Objektivbaugruppe 52 ist zwischen dem Deckglas 50 und einem Bildaufnehmer 54 angeordnet. Der Bildaufnehmer 54 bildet einen Bestandteil einer Bildaufnahmeeinheit. Der Bildaufnehmer 54 ist beispielhaft als Bildsensor, etwa als CCD-Sensor ausgebildet. Vom Bildaufnehmer 54 erfasste Bildsignale können als Rohdaten oder als hiervon abgeleitete Daten über eine Signalleitung 56 in Richtung auf das proximale Ende des Schaftes 14 übermittelt werden. Die Signalleitung 56 mündet in einen Gehäuseanschluss 58. Schlussendlich können die Bildsignale oder hiervon abgeleitete Daten über den Anschluss 22 ausgegeben werden.

Demgemäß ist das Instrument 10 zur elektronischen Bilderfassung geeignet (Video- Mediastinoskop bzw. Video-Endoskop). Es ist grundsätzlich auch vorstellbar, Instrumente 10 mit einem rein optischen Beobachtungspfad zu versehen, wobei der Beobachtungspfad beispielsweise einem Okular für die direkte Beobachtung zugeführt wird.

Die Beleuchtungseinheit 48 umfasst beispielhaft einen oder mehrere Lichtleiter in Form von Glasfasern 60. Die Beobachtungsoptik 46 (allgemein der Beobachtungspfad) und die Beleuchtungseinheit 48 (allgemein der Beleuchtungspfad) teilen sich den durch den zweiten Kanal 42 bereitgestellten Bauraum. Grundsätzlich erzeugt der Bildaufnehmer 54 bzw. allgemein die Bildaufnahmeeinheit im Betrieb eine gewisse Abwärme. Ferner muss auch bei der Beleuchtungseinheit 48 im Betrieb mit einem gewissen Wärmeeintrag in den Schaft 14 gerechnet werden. Dies kann die Belastung für den Patienten erhöhen. Deshalb ist es grundsätzlich wünschenswert, überschüssige Wärme, die im distalen Bereich des Schaftes 14 entsteht, in Richtung auf das proximale Ende 32 vom Schaft 14 abzuführen.

Zu diesem Zweck sieht die Ausführungsform gemäß Fig. 3 Kühlelemente 64, 66 vor, welche sich benachbart zum zweiten Kanal 42 im Grundkörper 16 erstrecken. Das Kühlelement 64 weist ein distales Ende 68 auf. Das Kühlelement 66 weist ein distales Ende 70 auf. Die distalen Enden 68, 70 sind dem distalen Ende des zweiten Kanals 42 benachbart. Demgemäß kann über die Kühlelemente 64, 66 Wärme aus diesem Bereich abgeführt werden.

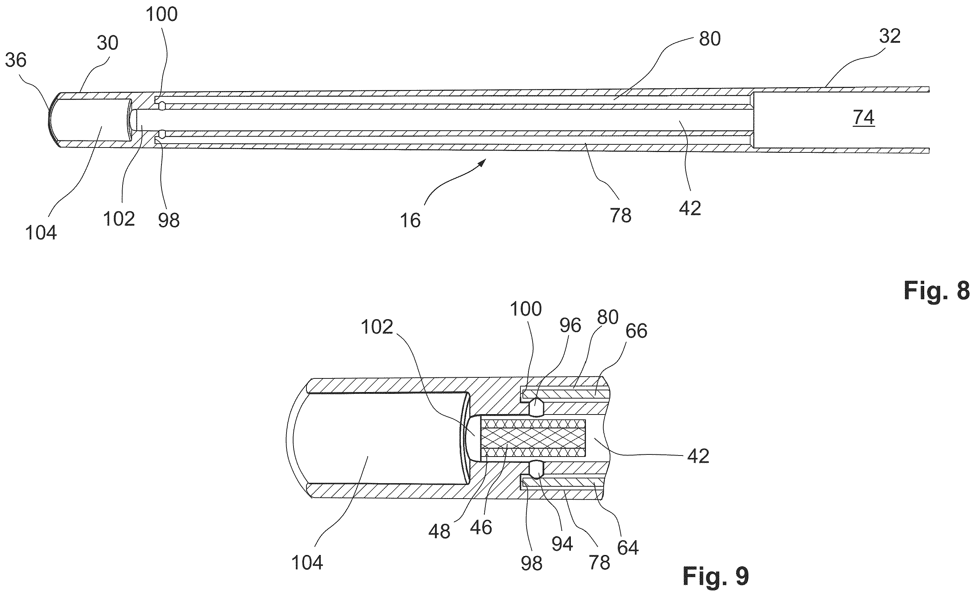

Am Grundkörper 16 bzw. am Schaft 14 ist ein Anschlussbereich 74 für das Verbindungsstück 18 ausgebildet, vergleiche Fig. 5 sowie Fig. 8. Der Anschlussbereich 74 ist an den Sitz 24 des Verbindungsstücks 18 angepasst, vergleiche hierzu Fig. 3.

Die in Fig. 3 veranschaulichten Kühlelemente 64, 66 sind in Nebenkanälen 78, 80 im Grundkörper 16 angeordnet, deren Gestaltung nachfolgend mit Bezugnahme auf die Figuren 5-9 veranschaulicht wird. Fig. 5 zeigt eine seitliche Längsansicht des Grundkörpers 16 auf diejenige Seite, bei der der Schlitz 34 angeordnet ist. In Fig. 5 veranschaulicht eine Linie VI-VI die Orientierung der Ansicht gemäß Fig. 6. Ferner veranschaulicht eine Linie VII-VII in Fig. 5 die Orientierung der Ansicht gemäß Fig. 7. Ferner veranschaulicht eine Linie VIII-VIII in Fig. 6 die Orientierung der Ansicht in Fig. 8. Einer Zusammenschau der Figuren 6-8 ist die grundsätzliche Gestaltung und Anordnung der Nebenkanäle 78, 80 im Grundkörper 16 entnehmbar.

Fig. 6 ist die Lage einer Längsmittelebene 82 durch den Schaft 14 bzw. den Grundkörper 16 entnehmbar. Fig. 5 veranschaulicht ferner eine Längsachse 84 des Schaftes 14. Die Längsachse 84 definiert eine Längserstreckung des Schaftes 14. Der Schaft 14 bzw. dessen Grundkörper 16 sind jedoch nicht streng rotationssymmetrisch zur Längsachse 84 ausgerichtet. Die Längsachse 84 erstreckt sich durch die Längsmittelebene 82.

Die Nebenkanäle 78, 80 sind im Ausführungsbeispiel symmetrisch zur Längsmittelebene 82 angeordnet. Zwischen den beiden Nebenkanälen 78, 80 ist der zweite Kanal 42 angeordnet. Eine Längsachse (nicht dargestellt) des zweiten Kanals 42 erstreckt sich durch die Längsmittelebene 82. Die Längsachsen (nicht dargestellt) der Nebenkanäle 78, 80 bilden gemeinsam eine Ebene, die im Wesentlichen senkrecht zur Längsmittelebene 82 liegt. Durch die benachbarte Anordnung der Nebenkanäle 78, 80 in Bezug auf den zweiten Kanal 42 ist eine effiziente Wärmeabfuhr möglich.

Fig. 6 und Fig. 7 veranschaulichen ferner einen sich ergebenden Querschnitt 86 des ersten Kanals 40. Der erste Kanal 40 hat einen nierenförmigen Querschnitt 86. Die längere Hauptachse des Querschnitts 86 ist senkrecht zur Längsmittelebene 82 orientiert. Die kürzere Hauptachse des Querschnitts 86 fällt mit der Längsmittelebene 82 zusammen.

Der nierenförmige Querschnitt 86 weist eine konkave "Delle" auf, welche innerhalb des den ersten Kanal 40 umgebenden Mantels 28 genügend Bauraum für die Einbringung des zweiten Kanals 42 sowie der Nebenkanäle 78, 80 schafft. Gleichwohl ist der nierenförmige Querschnitt 86 mit weichen, glatten Übergängen, vorzugsweise mit tangentialen Übergängen und ohne enge, scharfe Innenkanten gestaltet. Dies vereinfacht die Reinigung oder Aufbereitung des Schaftes 14.

In demjenigen Bereich des Mantels 28, in dem der zweite Kanal 42 ausgebildet ist, ist demgemäß eine Aufdickung 88 mit einer (konvexen) Erhebung 90 nach innen in den ersten Kanal 40 hinein vorgesehen. Auf diese Weise wird der konkave Abschnitt des nierenförmigen Querschnitts 86 gebildet. Die Aufdickung 88 stellt also genügend Wandstärke für den zweiten Kanal 42 bereit. Da glatte Übergänge bei der Innenkontur gewünscht sind, die den ersten Kanal 40 und dessen Querschnitt 86 ausbildet, ist beidseits des zweiten Kanals 42 genügend Bauraum vorhanden, um dort neben dem zweiten Kanal 42 die beiden Nebenkanäle 78, 80 für die Wärmeabfuhr auszubilden. Diese zusätzliche Funktion wird also nicht mit Bauraumnachteilen erkauft. Ganz im Gegenteil, dass Auffüllen potenzieller Innenkanten, welche bei der Reinigung problematisch sein können, bringt weitere Vorteile mit sich.

Der in Fig. 7 gezeigte Querschnitt durch den Grundkörper 16 veranschaulicht, dass sich im Bereich der Aufdickung 88 ein günstiges Widerstandsmoment ergibt, so dass der Grundkörper 16 insgesamt hinreichend steif ist. Dies ermöglicht eine durchgehende Gestaltung des Schlitzes 34, vergleiche Fig. 5.

Der Schaft 14 ist im gezeigten Ausführungsbeispiel insgesamt ausgehend vom proximalen Ende 32 in Richtung auf das distale Ende 30 leicht verjüngt. Dies kann dazu führen, dass die Längsachsen der Kanäle 40, 42, 78, 80 nicht unbedingt streng parallel zueinander ausgerichtet sein müssen. Es ist zumindest eine leichte Neigung zwischen den Kanälen 40, 42, 78, 80 vorstellbar, die an die Verjüngung des Schaftes 14 angepasst ist. Gleichwohl sind auch Gestaltungen mit streng paralleler Orientierung der Kanäle 40, 42, 78, 80 vorstellbar.

Mit ergänzender Bezugnahme auf die Schnittdarstellung gemäß Fig. 8 sowie die ergänzende Detailansicht des distalen Endes 30 gemäß Fig. 9 wird die funktionale Kopplung zwischen den Kanälen 42, 78, 80 weiter veranschaulicht. Zwischen dem Nebenkanal 78 und dem zweiten Kanal 42 ist ein Verbindungskanal 94 vorgesehen. Gleichermaßen ist zwischen dem Nebenkanal 80 und dem zweiten Kanal 42 ein Verbindungskanal 96 vorgesehen. Die Verbindungskanäle 94, 96 bilden eine Querverbindung zwischen den Nebenkanälen 78, 80 und dem zweiten Kanal 42.

Der zweite Kanal 42 erstreckt sich durch den Grundkörper 16 hindurch und verbindet somit das distale Ende 30 und das proximale Ende 32. Die Nebenkanäle 78, 80 sind hingegen - zumindest axial betrachtet - als Blindlöcher bzw. Sacklöcher gestaltet. Diese Gestaltung wird durch die Bezugszeichen 98, 100 veranschaulicht, welche jeweilige Sacklöcher am distalen Ende der Nebenkanäle 78, 80 bezeichnen. Hingegen gibt es beim zweiten Kanal 42 einen Durchgang 102, der in eine Mulde 104 beim distalen Ende 30 des Grundkörpers 16 mündet. Die Mulde 104 kann auch als Auslauf des ersten Kanals 40 bezeichnet werden.

Die Verbindungskanäle 94, 96 optimieren die Wärmeabfuhr, da über die Verbindungskanäle 94, 96 Wärme gezielt vom distalen Ende des zweiten Kanals 42 zu den Kühlelementen 64, 66 in den Nebenkanälen 78, 80 abgeführt werden kann. Über die Verbindungskanäle 94, 96 sind die Nebenkanäle 78, 80 mit einem Bereich des zweiten Kanals 42 gekoppelt, in dem mit einer erhöhten Wärmeerzeugung zu rechnen ist.

Zur Verbesserung der Wärmeabführung ist es vorstellbar, die Verbindungskanäle 94, 96 und generell die "Lücke" zwischen den Kühlelementen 64, 66 und der Beobachtungsoptik 46 mit dem Bildaufnehmer 54 bzw. der Beleuchtungseinheit 48 durch geeignete Stoffe (Wärmeleitpaste o. ä.) zu überbrücken. Allgemein können die Verbindungskanäle 94, 96 mit einem Stoff mit hoher Wärmeleitfähigkeit gefüllt sein.

Die Verbindungskanäle 94, 96 können mit konventionellen, abtragenden Herstellverfahren nicht oder nur mit hohem Aufwand hergestellt werden. Insbesondere dann, wenn der Grundkörper 16 einstückig gestaltet sein soll, ist eine konventionelle Erzeugung der Verbindungskanäle 94, 96 nahezu unmöglich. Die additive Fertigung erlaubt hingegen eine derart unkonventionelle Gestaltung.

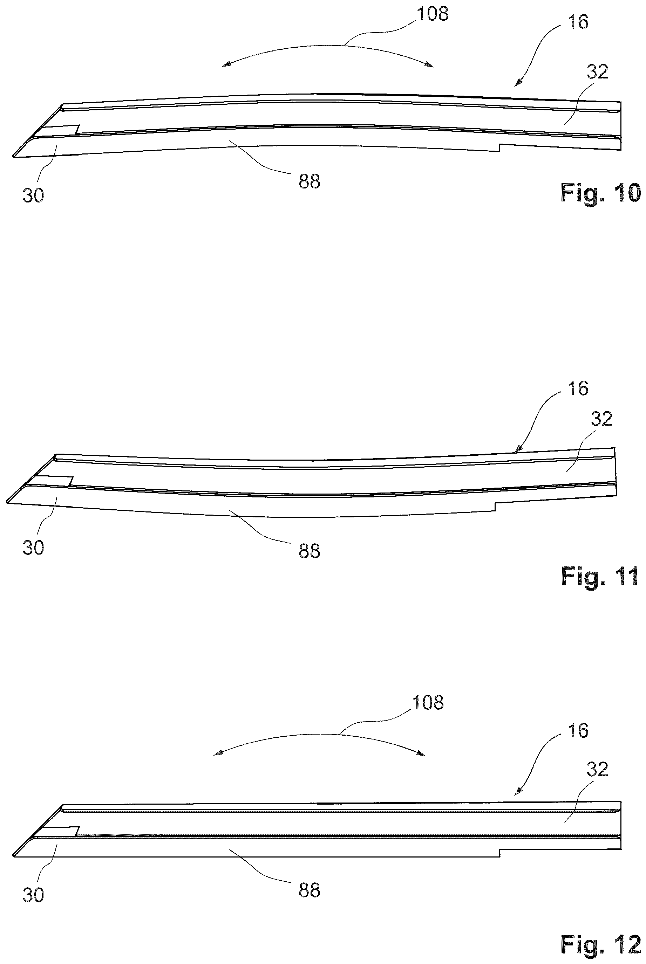

Mit Bezugnahme auf die Figuren 10-12 wird anhand vereinfachter seitlicher Längsansichten des Grundkörpers 16 ein Ansatz zum Umgang mit fertigungsbedingtem Verzug bei der additiven Fertigung des Grundkörpers 16 veranschaulicht. Bei der additiven Fertigung ist prinzipbedingt mit einer gewissen Deformation (Verzug) der Bauteile zu rechnen. Insbesondere bei Bauteilen mit inhomogener/ungleichmäßiger Materialverteilung muss aufgrund entsprechend inhomogener Schrumpfung mit Verzug gerechnet werden. Es versteht sich, dass die Krümmungen bzw. der Verzugsgrad der in den Figuren 10-12 gezeigten Bauteile aus Veranschaulichungsgründen übertrieben sein kann.

Fig. 10 zeigt einen sich ergebenden Verzug bei dem Grundkörper 16, wenn die der Fertigung zugrunde liegende Datenverkörperung (CAD-Modell) ideal gerade ausgerichtet ist, vergleiche hierzu die Darstellung in Fig. 5 mit der geraden Längsachse 84. Es versteht sich, dass ein Verzug nicht nur in der gezeigten Ebene präsent sein kann, sondern auch in einer Ebene senkrecht zur Ansichtsebene.

Ursache des Verzugs ist beispielsweise die die Aufdickung 88 bildende Materialanhäufung, vergleiche hierzu auch Fig. 7. Wenn dieser Bereich tendenziell stärker "schrumpft" als dünnere Bereiche des Mantels 28, dann kann sich nach der Fertigung und dem Abkühlen die in Fig. 10 gezeigte gekrümmte Gestalt ergeben. Die Tendenz der Deformation wird durch den mit 108 bezeichneten gekrümmten Doppelpfeil veranschaulicht.

Um derartige Deformationen zu vermeiden, wird daher vorgeschlagen, den Verzug bei der Datenverkörperung (der Fertigung zugrundeliegendes CAD-Modell) vorzuhalten. Beispielsweise entspricht die für die Fertigung genutzte Datenverkörperung der in Fig. 11 gezeigten Gestaltung. Mit anderen Worten ist der Grundkörper 16 in der Datenverkörperung entgegengesetzt zur erwarteten verzugsbedingten Krümmung (vergleiche Fig. 10) gekrümmt. Dies führt nun nach der Fertigung und dem Abkühlen im Ausführungsbeispiel zu der in Fig. 12 gezeigten Gestaltung. Im Idealfall führt dann die unvermeidbare Krümmung dazu, dass ausgehend von der vorgehaltenen, entgegensetzen Krümmung in Fig. 11 ein gerader oder nahezu gerader Zustand erreicht wird, vergleiche wiederum den Doppelpfeil 108 zur Veranschaulichung der Tendenz der fertigungsbedingten Deformation.

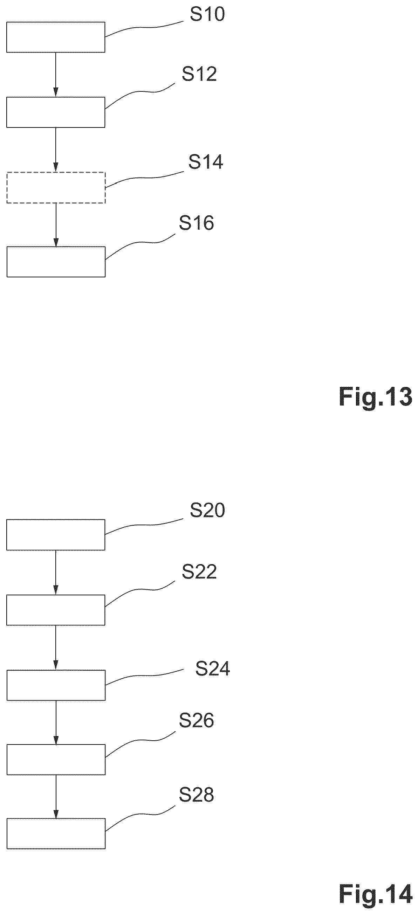

Mit Bezugnahme auf Fig. 13 wird anhand eines Blockdiagramms eine beispielhafte Ausgestaltung eines Verfahrens zur Herstellung eines Bauteils für ein medizinisches Instrument, insbesondere eines schaftförmigen Spatels mit mehreren Kanälen, veranschaulicht. Das Verfahren umfasst einen Schritt S10, der die Bereitstellung einer (CAD-) Datenverkörperung umfasst. Die Datenverkörperung kann auf Basis eines CAD-Modells generiert werden. Die Datenverkörperung wird mittelbar oder unmittelbar einer Anlage zur additiven Fertigung zugeführt, um dort auf dieser Basis den den Schaft bildenden Grundkörper einstückig und integral auszubilden, Schritt S12. Insbesondere erfolgt die additive Fertigung unter Verarbeitung eines Metallwerkstoffes, beispielsweise eines Edelstahlpulvers. Der Grundkörper umfasst zumindest einen ersten Kanal, einen zweiten Kanal und zumindest einen Nebenkanal, der dem zweiten Kanal benachbart und mit diesem verbunden ist. Vorzugsweise wird der Grundkörper auf diese Weise nachbearbeitungsarm oder sogar nachbearbeitungsfrei erzeugt.

Es kann sich ein optionaler Schritt S14 anschließen. Der Schritt S14 umfasst eine Nachbearbeitung, insbesondere eine Oberflächenbearbeitung zumindest eines der Kanäle mittels Strömungsschleifen. Auf diese Weise kann etwa im zweiten Kanal oder im ersten Kanal eine gewünschte Oberflächenqualität erzeugt werden.

Schließlich folgt ein Schritt S16, der ein Fügen des Schaftes mit einem Griff umfasst. Dies kann unter Zwischenschaltung eines Verbindungsstückes erfolgen. Auf diese Weise kann insgesamt mit wenigen Schritten ein Instrument mit komplex gestaltetem Schaft hergestellt werden. Dies kann etwa Instrumente in Form von Mediastinoskopen oder allgemein endoskopische Instrumente betreffen.

Mit Bezugnahme auf Fig. 14 wird anhand eines Blockdiagramms eine weitere beispielhafte Ausgestaltung eines Verfahrens zur Herstellung eines Bauteils für ein medizinisches Instrument, insbesondere eines schaftförmigen Spatels mit mehreren Kanälen, veranschaulicht. Die in Fig. 14 gezeigte Ausgestaltung ergänzt das anhand der Fig. 13 veranschaulichte Verfahren. Die Verfahrensschritte gemäß Fig. 14 behandeln insbesondere eine Herstellung des Instruments, insbesondere von dessen Schaft, mit Kompensation des fertigungsbedingten Verzugs.

Das Verfahren umfasst einen Schritt S20, der die Bereitstellung einer idealen Datenverkörperung umfasst. Dabei handelt es sich beispielsweise um ein CAD-Modell, welches einen etwaigen Verzug noch nicht berücksichtigt. D. h. mit anderen Worten, gäbe es keinen Verzug oder sonstige Deformationen bei der Fertigung, würde sich bei einer 1:1 Umsetzung der idealen Datenverkörperung die gewünschte Gestalt ergeben.

Jedoch muss bei der additiven Fertigung mit einer gewissen Schrumpfung und infolgedessen mit einem gewissen Verzug gerechnet werden. Daher folgt auf den Schritt S20 ein weiterer Schritt S22, der eine Vorhersage des zu erwartenden Verzuges umfasst. Der Schritt S22 kann Berechnungen oder Simulationen umfassen, jedoch auch Versuche mit realen Bauteilen oder kombinierte Ansätze. Auf Basis der Vorhersage im Schritt S22 wird in einem weiteren Schritt S24 ein angepasstes Modell des Bauteils, also eine angepasste Datenverkörperung erzeugt und bereitgestellt. Die Datenverkörperung nimmt den zu erwartenden Verzug vorweg. Mit anderen Worten enthält die Datenverkörperung beispielsweise eine Geometrie, die entgegengesetzt zur erwarteten Krümmung gekrümmt ist.

Sodann folgt in einem Schritt S26 die additive Herstellung des Bauteils, vergleiche den Schritt S12 in Fig. 13. Unmittelbar nach der additiven Herstellung (bzw. sogar mit zeitlicher Überlappung zu dieser) schließt sich ein Schritt S28 an, der die verfahrensbedingte Schrumpfung und den verfahrensbedingten Verzug am realen Bauteil enthält. Da die Fertigung auf Basis des angepassten Modells erfolgt, ergibt sich im Schritt S28 eine Geometrie, die der idealen Gestalt (vergleiche Schritt S20) weitgehend oder vollständig entspricht.

## Patentansprüche

1. Medizinisches Instrument zur Bereitstellung eines Zugangs zum Körperinneren, insbesondere Spatel (12) für ein endoskopisches Instrument (10), das Folgendes aufweist:
- einen Schaft (14) mit einem langgestreckten Grundkörper (16),
wobei sich der Grundkörper (16) zwischen einem distalen Ende (30) und einem proximalen Ende (32) des Schaftes (14) erstreckt,
- einen mit dem Grundkörper (16) des Schaftes (14) an dessen proximalem Ende (32) gekoppelten Griff (20),
wobei der Schaft (14) zumindest einen ersten Kanal (40) und einen zweiten Kanal (42) definiert, die sich durch den Schaft (14) erstrecken,
wobei der erste Kanal (40) eine Passage für chirurgische Instrumente durch den Schaft (14) hindurch bereitstellt,
wobei der zweite Kanal (42) zur Aufnahme einer Beobachtungsoptik (46) ausgebildet ist, und
wobei dem zweiten Kanal (42) zumindest ein Nebenkanal (78, 80) benachbart ist, der mit dem zweiten Kanal (42) verbunden ist.

2. Instrument nach Anspruch 1,
wobei der Grundkörper (16) des Schaftes (14) integral gestaltet und vorzugsweise durch ein additives Fertigungsverfahren erzeugt ist.

3. Instrument nach Anspruch 1 oder 2,
wobei der Grundkörper (16) zumindest abschnittsweise, vorzugsweise vollständig zwischen dem distalen Ende (30) und dem proximalen Ende (32), entlang seiner Längserstreckung geschlitzt ist.

4. Instrument nach einem der Ansprüche 1 bis 3,
wobei der zweite Kanal (42) neben der Beobachtungsoptik (46) ferner eine Beleuchtungseinheit (48) beherbergt, und
wobei der zweite Kanal (42) vorzugsweise in seinem distalen Endbereich eine Objektivbaugruppe (52) beherbergt, die einem Bildaufnehmer (54) vorgelagert ist.

5. Instrument nach einem der Ansprüche 1 bis 4,
wobei der zweite Kanal (42) über einen Verbindungskanal (94, 96) mit dem zumindest einen Nebenkanal (78, 80) verbunden ist, und
wobei der Verbindungskanal (94, 96) vorzugsweise in einem distalen Endbereich oder benachbart zu diesem angeordnet ist.

6. Instrument nach einem der Ansprüche 1 bis 5,
wobei der zumindest eine Nebenkanal (78, 80) als Sackloch gestaltet und am distalen Ende (30) des Schaftes (14) geschlossen ist.

7. Instrument nach einem der Ansprüche 1 bis 6,
wobei der zumindest eine Nebenkanal (78, 80) ein Wärmeabfuhrelement beherbergt, um Wärme vom distalen Ende (30) hin zum proximalen Ende (32) abzuführen.

8. Instrument nach einem der Ansprüche 1 bis 7,
wobei der zweite Kanal (42) zwischen zwei Nebenkanälen (78, 80) angeordnet ist, die einen distalen Endbereich des zweiten Kanals (42) mit dem proximalen Ende (32) des Instruments verbinden.

9. Instrument nach einem der Ansprüche 1 bis 8,
wobei der erste Kanal (40) einen nierenförmigen Querschnitt (86) aufweist, und wobei der zweite Kanal (42) einem konkaven Abschnitt (90) des nierenförmigen Querschnitts benachbart ist.

10. Instrument nach einem der Ansprüche 1 bis 9,
wobei der zweite Kanal (42), bei Betrachtung einer gedachten Längsmittelebene (82) durch den Schaft (14), zwischen dem ersten Kanal (40) und dem Griff (20) angeordnet ist, und
wobei der zumindest eine Nebenkanal (78, 80) seitlich der gedachten Längsmittelebene (82) durch den Schaft (14) angeordnet ist.

11. Instrument nach einem der Ansprüche 1 bis 10,
wobei der Grundkörper (16) des Schaftes (14) an seinem Umfangsbereich, der dem Griff (20) zugewandt ist, eine Materialanhäufung (88) aufweist, welche den zweiten Kanal (42) und die zumindest einen Nebenkanal (78, 80) beherbergt.

12. Instrument nach einem der Ansprüche 1 bis 11,
wobei sich der Grundkörper (16) ausgehend vom proximalen Ende (32) in Richtung des distalen Endes (30) zumindest abschnittsweise verjüngt, vorzugsweise kontinuierlich verjüngt, und
wobei vorzugsweise am distalen Ende (30) des Schaftes (14) beim Grundkörper (16) eine Spitze (36) ausgebildet ist, die insbesondere durch einen schrägen Abschnitt des Grundkörpers (16) gebildet ist.

13. Verfahren zur Herstellung eines medizinischen Instruments zur Bereitstellung eines Zugangs zum Körperinneren, insbesondere eines Spatels (12) für ein endoskopisches Instrument (10), wobei das Verfahren die folgenden Schritte aufweist:
- Bereitstellung einer Datenverkörperung eines Schaftes (14) mit einem langgestreckten Grundkörper (16),
wobei sich der Grundkörper (16) zwischen einem distalen Ende (30) und einem proximalen Ende (32) des Schaftes (14) erstreckt,
wobei der Schaft (14) zumindest einen ersten Kanal (40) und einen zweiten Kanal (42) definiert, die sich durch den Schaft (14) erstrecken,
wobei der erste Kanal (40) eine Passage für chirurgische Instrumente durch den Schaft (14) hindurch bereitstellt,
wobei der zweite Kanal (42) zur Aufnahme einer Beobachtungsoptik (46) ausgestaltet ist, und
wobei dem zweiten Kanal (42) zumindest ein Nebenkanal (78, 80) benachbart ist, der mit dem zweiten Kanal (42) verbunden ist,
- integrale Fertigung des Schaftes (14) mit dem ersten Kanal (40), dem zweiten Kanal (42) und dem zumindest einen Nebenkanal (78, 80) in einem additiven Fertigungsverfahren auf Basis eines pulverförmigen metallischen Ausgangsmaterials, insbesondere eines austenitischen, rostfreien Stahlwerkstoffs, unter Berücksichtigung der Datenverkörperung, und
- Verbindung des Schaftes (14) mit einem Griff (20) am proximalen Ende (32) des Grundkörpers (16) des Schaftes (14).

14. Verfahren nach Anspruch 13,
wobei der Schritt der Fertigung die Nutzung einer Pulverbett basierten Vorrichtung zur additiven Fertigung umfasst, und
wobei Pulver in einem Bauraum der Vorrichtung durch energiereiche Strahlung erweicht und gefügt wird.

15. Verfahren nach Anspruch 13 oder 14,
wobei der Schritt der Bereitstellung der Datenverkörperung eine Bereitstellung einer einen erwarteten Verzug des Bauteils bei der Fertigung vorwegnehmenden Datenverkörperung umfasst, und/oder
wobei ferner zumindest ein abtragender Nachbearbeitungsschritt vorgesehen ist, der ein Strömungsschleifen zumindest des zweiten Kanals (42) umfasst.
